# EUROPÄISCHE PATENTSCHRIFT

(11) **EP 1 888 540 B1**
(45) Veröffentlichungstag und Bekanntmachung des Hinweises auf die Patenterteilung: **18.06.2008**
(21) Anmeldenummer: 06707437.7
(22) Anmeldetag: 06.03.2006
(51) Int. Cl.: C07D 251/60

(54) **VERFAHREN ZUM HERSTELLEN VON MELAMIN MIT WÄRMERÜCKGEWINNUNG**
METHOD FOR PRODUCING MELAMINE WITH HEAT RECOVERY
PROCEDE DE PRODUCTION DE MELAMINE AVEC RECUPERATION DE CHALEUR

(30) Priorität: 06.05.2005 DE 102005021082; 13.05.2005 DE 102005023041
(43) Veröffentlichungstag der Anmeldung: 20.02.2008
(73) Patentinhaber: Lurgi AG, 60295 Frankfurt am Main (DE)
(72) Erfinder: MÜLLER-HASKY, Martin, 63150 Heusenstamm (DE); EBERHARDT, Jürgen, 63110 Rodgau (DE); SCHADT, Arne, 61231 Bad Neuheim (DE)
(74) Vertreter: Meyer-Dulheuer, Karl-Hermann
(86) Internationale Anmeldenummer: PCT/EP2006/002019
(87) Internationale Veröffentlichungsnummer: WO 2006/119815

(56) Entgegenhaltungen:
- US-A- 4 348 520
- G.M. CREWS, W. RIPPERGER, D.B. KERSEBOHM, J. SEEHOLZER, T. GÜTHNER: "Melamine and Guanamines" ULLMANN'S ENCYCLOPEDIA OF INDUSTRIAL CHEMISTRY, WILEY, VCH VERLAG, WEINHEIM, DE, Bd. 21, 2003, Seiten 205-216, XP002393510

## Beschreibung

Die Erfindung betrifft ein Verfahren zur Herstellung von Melamin durch Zersetzung von Harnstoff in einem Wirbelschichtreaktor wobei das heiße Reaktionsgas in einem Gaskühler abgekühlt wird und die hierbei gewonnene Wärme zur Vorheizung des für die Erzeugung der Wirbelschicht benötigte Wirbelgas direkt genutzt wird.

Die Herstellung von Melamin ausgehend von Harnstoff ist ein lange bekanntes Verfahren, wobei zwei Arten von Prozessführung zu unterscheiden sind: das nicht katalytische Hochdruckverfahren und das katalytische Niederdruckverfahren. Das Hochdruckverfahren erfordert Drucke von mindestens 8 MPa, das katalytische Niederdruckverfahren wird hingegen in einer Wirbelschicht bei einem Druck von maximal 1 MPa und Temperaturen von mindestens 380 bis 410°C durchgeführt. Das verwendete Trägergas beim Niederdruckverfahren besteht entweder aus Ammoniak oder aus einer Mischung aus Kohlendioxid und Ammoniak, wobei das erhaltene Melamin nach der Reaktion in gasförmiger Form vorliegt.

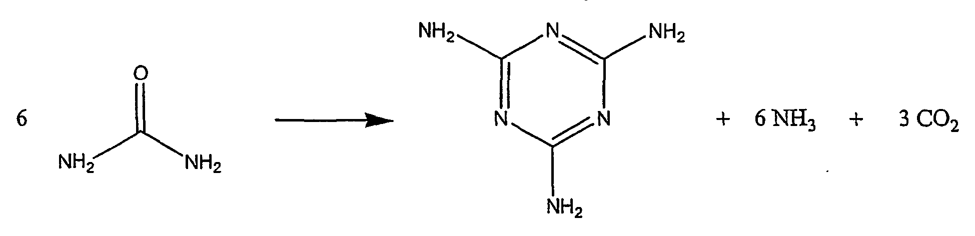

Die Reaktion von Harnstoff zu Melamin ist endotherm, so dass dem System von außen große Wärmemengen zur Verfügung gestellt werden müssen.

Die Ausbeute an Melamin beträgt so 90 bis 95%, bezogen auf die eingesetzte Menge an Harnstoff. In der Literatur sind die drei häufigsten Niederdruckverfahren bekannt als BASF-Verfahren, Chemie-Linz-Verfahren und Stamicarbon-Verfahren.

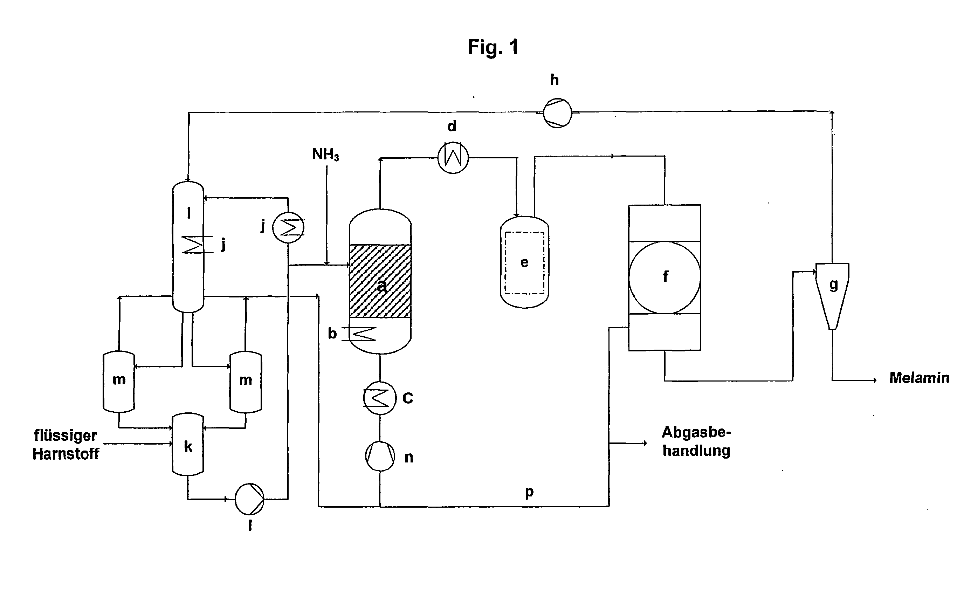

Bei dem BASF-Verfahren handelt es sich um ein einstufiges Reaktionsverfahren (Fig. 1), bei dem geschmolzener Harnstoff bei einer Temperatur von 395 bis 400°C unter nahezu atmosphärischem Druck in einer Wirbelschicht umgesetzt wird. Das hierbei erhaltene Reaktionsgas enthält neben Melamin zudem Spuren von Nebenprodukten wie Melem und Melam sowie Reaktionsgas bestehend aus Ammoniak und Kohlendioxid. Das entstandene Reaktionsgasgemisch wird anschließend abgekühlt, der ausgetragene Katalysator und die auskristallisierten Nebenprodukte abgetrennt und das melaminhaltige Reaktionsgas in einen Kristallisator eingespeist. In Kristallisator wird das melaminhaltige Gas mit Reaktionsgas, abgekühlt, wodurch die Temperatur des melaminhaltigen Gases auf Temperaturen von 190 bis 210°C abgekühlt wird. Unter diesen Bedingungen desublimiert Melamin zu annähernd 98% aus dem Reaktionsgas.. Nach der Abtrennung des Melamins wird das verbleibende Gas (Kreislaufgas) mit Hilfe eines Kreisgagebläses zu einer Harnstoffwäsche gefördert, wo es im direkten Kontakt mit der Harnstoffschmelze gekühlt und gewaschen wird. Die Temperatur des Quenchgases liegt bei - 138°C, sodass es zur Einstellung der Temperatur von 190 bis 220°C in der Kristallisationsvorrichtung erforderlich ist, pro Kilogramm melaminhaltiges Gas 2,5 bis 3,5 kg Quenchgas beizumengen.

Die Herstellung von Melamin ist ein seit langem bekannter und ausgereifter Prozess. So beschreibt bereits die deutsche Offenlegungsschrift DE 33 02 833 ein Verfahren zur katalytischen Herstellung von Melamin durch die thermische Umwandlung von Harnstoff. Bei diesem Verfahren werden die Synthesegase, aus denen das Melamin bereits abgeschieden ist, mit einer Harnstoffschmelze behandelt, wobei die Behandlung in einem Wäscher erfolgt.

Problematisch ist jedoch zum einen, dass aufgrund der endothermen chemischen Reaktion große Wärmemengen erforderlich sind, um den Wirbelschichtreaktor bei der erforderlichen Reaktionstemperatur von 395 bis 400°C zu halten und zum anderen, dass die Abkühlung des den Wirbelschichtreaktor verlassenden melaminhaltigen Reaktionsgases zur Abscheidung von unerwünschten Nebenprodukten, wie beispielsweise Melem oder Melam zwingend erforderlich ist. Die Abkühlung des melaninhaltigen Gases ist nicht unproblematisch. Der Beginn der Desublimation ist Abhängig von dem Druck und dem Gehalt an Melamin in dem Reaktor verlassenden Gas. Erreicht der Partialdruck den Sättigungsdampfdruck des Melamins wird folglich Melamin auskristallisieren. Es ist daher zwingend notwendig, dass der Kühler, der dem Reaktor folgt, das Gas nicht zu sehr abkühlt und schon dort eine Desublimation erfolgen lässt. Es muss sogar darauf geachtet werden, dass in dem Kühler selbst keine Stellen (d.h. Rohrwand) unterhalb des Desublimationstemperatur liegt, da sonst Melamin auskristallisieren würde. Diese Melaminanhaftungen führen zu deutlich verkürzten Anlagenbetriebszeiten, zu unerwünschtem Produktionsausfall und erhöhtem Wartungsbedarf. Um diese Anhaftungen zu verhindern wird die Wärme mit Hilfe eines speziellen Wärmeübertragungsmediums (Thermalöl) abgeführt, dass bei einer Temperatur arbeitet, bei der die kritische Rohrwandtemperatur des Kühlers nicht unterhalb der Desublimationstemperatur des Melamins fällt. Bei den Thermalölen handelt es sich um höher molekulare Kohlenwasserstoffe (z.B. Phenyle, ihre Derivate und Gemische), die bei Freisetzung gravierende Auswirkungen auf Mensch und Umwelt sowie Sachgegenstände haben können (toxisch, feuergefährlich). Das Thermalöl wiederum muss in einem nachgeschalteten Kühler oder Kondenstor behandelt werden.

Somit liegt der vorliegenden Erfindung das Problem zugrunde, eine Verfahren bereitzustellen, das er erlaubt, die erforderliche Reaktionstemperatur von 395 bis 400°C im Wirbelschichtreaktor auf eine kostengünstige und somit wirtschaftliche Art und Weise bereitzustellen und gleichzeitig die Reaktionswärme aus dem melaminhaltigen Reaktionsgas abzuführen, ohne dabei ein in seinen Eigenschaften kritisches Wärmeübertragungsmedium einzusetzen. Gleichzeitig soll die kritische Rohrwandtemperatur so eingestellt werden, dass eine unerwünschte vorzeitige Desublimation an Melamin im Kühler verhindert wird.

Diese Aufgabe wird durch das erfindungsgemäße Verfahren zur Herstellung von Melamin durch die Zersetzung von Harnstoff in einem Wirbelschichtreaktor gelöst, indem das heiße Reaktionsgas in einem Gaskühler abgekühlt wird und die hierbei gewonnene Wärme zur Vorheizung der für die Erzeugung der Wirbelschicht benötigte Gas übertragen wird. Dies konnte darin gefunden werden, dass das Gas, das zur Fluidisierung der Wirbelschicht benötigt wird, unmittelbar als Kühlmedium in dem Reaktor nachgeschalteten Kühler eingesetzt wird.

Die mit der Erfindung erzielten Vorteile bestehen darin, dass es durch das erfindungsgemäße Verfahren möglich ist, die Temperatur des melaminhaltigen Reaktionsgases auf die Temperaturen abzusenken, die eine Abtrennung von unerwünschten Nebenprodukten, wie Melem und Melam, gestatten. Gleichzeitig wird die hierbei gewonnene Wärmemenge zur Aufheizung des Wirbelgases verwendet. Durch das erfindungsgemäße Verfahren ist es möglich, einen Teil der benötigte Reaktionswärme in kostengünstiger Weise zurückzugewinnen, auf ein zusätzliches Wärmeübertragungsmedium und den zugehörigen Ausrüstungsgegenständen (Pumpen, Lagerbehälter, Kondensator bzw. Kühler) zu verzichten. Ferner können erhebliche Mengen an Primäbrennstoff eingespart werden, da die Wärmemenge, die zur Deckung der endothermen Reaktion benötigt wird, stark reduziert werden kann.

Vorteilhafte Ausgestaltungen der Erfindungen sind in den Patentansprüchen 2 und folgenden dargelegt. Gemäß Patentanspruch 2 ist es möglich, die Reaktionsgase in dem Gaskühler von 380 bis 420°C auf 210 bis 300°C abzukühlen. Gemäß Anspruch 3 ist es zudem möglich, die Wärmeübertragung in einem rohr- und/oder plattenförmigen Wärmeaustauscher erfolgen zu lassen. Eine weitere Ausgestaltung nach Anspruch 4 gestattet es, dass die Medien in dem Wärmeaustauscher entweder im Gleichstrom- oder Gegenstromprinzip strömen. Gemäß Anspruch 5 ist es möglich, eine Umführungsleitung (by-pass) zur Temperaturregelung einzusetzen.

Ausführungsbeispiele der Erfindung sind in den Zeichnungen dargestellt und werden in Folgenden näher beschrieben.

Es zeigen:
- Fig.1: Verfahren zur Herstellung von Melamin nach dem BASF-Verfahren;
- Fig. 2: erfindungsgemäße Verfahren zur Herstellung von Melamin mit Wärmerückgewinnung.

Fig. 1 zeigt das Verfahren zur Herstellung von Melamin nach dem BASF-Verfahren. Hierbei wird geschmolzener Harnstoff aus einem Harnstoffvorratsbehälter k dem System zugeführt. Durch eine Pumpe I wird der Harnstoff der Harnstoffwäsche i zugeleitet; in einem nachgeschalteten Tropfenabscheider m werden anschließend die flüssigen und die gasförmigen Bestandteile voneinander getrennt. Ein Teil der hierbei erhaltenen gasförmigen Bestandteile werden durch einen Kompressor n verdichtet, in einem Heizelement c vorgeheizt und anschließend dem Reaktor a zugeführt, wo sie zur Ausbildung der katalytisch wirksame Wirbelschicht benötigt werden. Die den Reaktor a verlassenden gasförmigen Bestandteile werden durch einen Gaskühler d gekühlt und anschließend einem Gasfilter e zugeleitet. In diesem Gasfilter e werden ausgefallene Nebenprodukte, wie zum Beispiel Melem, Melam und Katalysatoraustrag von dem Reaktionsgas getrennt. Das erhaltene Reaktionsgas wird anschließend einer Kristallisationsvorrichtung f zugeführt, wo dieses mit weiterem Reaktionsgas (Quenchgas), welches vorzugsweise eine Temperatur von ca. 140°C aufweist, auf Temperaturen von 190 bis 210°C gekühlt wird. Das in der Kristallisationsvorrichtung erhaltene kristalline Melamin wird anschließend einem Produktzyklon g zugeführt, im dem der Gasstrom von dem kristallinen Melamin abgetrennt wird. Nach Abtrennung des Melamins wird das Gas mit Hilfe eines Kreisgasgebläses h zur Harnstoffwäsche i befördert.

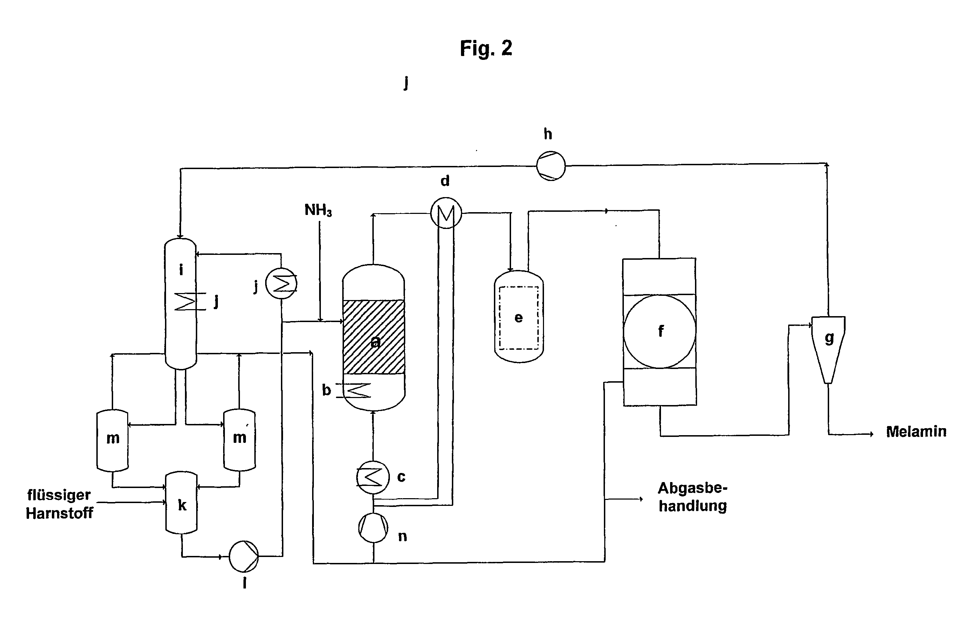

Fig. 2 zeigt ein erfindungsgemäßes Verfahren zur Herstellung von Melamin mit Wärmerückgewinnung. Flüssiger Harnstoff wird hierbei durch einen Harnstoffvorratsbehälter k dem System zugeführt. Durch eine Pumpe I und Kühleinheit j wird der flüssige Harnstoff der Harnstoffwäsche i zugeführt, wobei anschließend in einem Tropfenabscheider m flüssige und gasförmige Bestandteile voneinander getrennt werden. Die gasförmigen Bestandteile werden anschließend durch einen Kompressor n verdichtet. Die zur Ausbildung der katalytisch wirkenden Wirbelschicht benötigte Gas wird nun in einem Gaskühler d vorgewärmt. Dabei kühlt es das aus dem Reaktor kommende heiße und melaminhaltige Reaktionsgas. Anschließend durchströmt das vorgewärmte Fluidisierungsgas ein weiteres Gasheizelement c, um es annähernd auf Temperaturen von 400°C aufzuheizen; die erhitzten Gase werden anschließend dem Reaktor a zugeführt. Die im Reaktor gebildeten gasförmigen Bestandteile werden, nach dem sie in dem Gaskühler d gekühlt wurden, einem Gasfilter e zugeführt, in dem auskristallisierte Bestandteile wie Melem, Melam und Katalysatoraustrag von den gasförmigen Bestandteilen getrennt werden. Anschließend werden die Reaktionsgase in eine Kristallisationsvorrichtung f eingebracht, wo dieses mit weiterem Reaktionsgas (Quenchgas), welches vorzugsweise eine Temperatur von ca. 140°C aufweist, auf Temperaturen von 190 bis 210°C gekühlt. Das hierbei gebildete Melamin wird anschließend durch einen Produktzyklon g von den gasförmigen Bestandteilen abgetrennt. Das Gas strömt mit Hilfe des Kreisgasgebläses h zurück zum Harnstoffwäscher. Dort werden die Gase mit flüssigem Harnstoff gekühlt und gewaschen.

Die Erfindung wird anhand der Beispiele 1 und 2 näher beschrieben.

### Beispiel 1:

Beispiel 1 beschreibt die herkömmliche Kühlung des Gases mit verdampfendem Thermalöl. Der den Reaktor a verlassende heiße, melaminhaltige Gasstrom hat bei einer Temperatur von 400°C und einem Druck von 0,17 MPa (abs.) die folgende Zusammensetzung.

| | **kmol/h** | **mol-%** |
|---|---|---|
| Ammoniak NH₃ | 1709 | 67 |
| Kohlendioxid CO₂ | 740 | 29 |
| Melamin C₃H₆N₆ | 51 | 2 |
| Isocyansäure HNCO | 38 | 1,5 |
| Inert | 31 | 0,5 |
| Summe | 2551 | 100 |

Der Partialdruck des Melamins beträgt folglich 0,0034 MPa. Die zu diesem Druck gehörende Sättigungstemperatur beträgt ca. 310°C. Somit darf das Gas in dem nachgeschalteten Kühler d nicht kälter als 310°C werden bzw. darf an keiner Stelle im Kühler die Temperatur auf 310°C abfallen, da sonst Melamin auskristallisieren würde. Das verdampfende Thermalöl wird auf der Mantelseite eines Rohrbündelwärmetauschers geführt. Aufgrund des sehr hohen Wärmeübergangs auf der Mantelseite wird die Rohrinnenwandtemperatur annähernd die Temperatur des verdampfenden Thermalöls einnehmen. Somit muss die Verdampfungstemperatur des Thermalöls auf mindestens 315 °C eingestellt werden. Dagegen wird der Wärmedurchgangskoeffizient (k-Zahl), der zusammen mit der mittleren logarithmischen Temperaturdifferenz die Fläche eines Wärmeaustauschers bestimmt, immer kleiner sein als der kleinste Wärmeübergangskoeffizienten (Alphawert). Der kleinste Alphawert findet sich auf der gasdurchströmten Rohrseite und ist erfahrungsgemäß sehr klein. Er beträgt in diesem Beispiel ca. 160 W/m^{2°}C. Das Gas - aus dem Reaktor kommend - wird im Kühler auf ca. 340°C abgekühlt. Es ergibt somit zwischen dem Gas und dem Thermalöl eine mittlere logarithmische Temperaturdifferenz von ca. 40°C.

### Beispiel 2:

Das Gas aus dem Reaktor a mit der gleichen Zusammensetzung und gleichem Druck und Temperatur wie in Beispiel 1 wird in einem nachgeschalteten Kühler auf 340°C abgekühlt. Als Kühlmedium wird das Gas benutzt, das zur Fluidisierung der Wirbelschicht benötigt wird. Dazu führt man das Gas vom Kompressor kommend auf der Mantelseite eines Rohrbündelwärmetauschers. Dabei wird sich das verdichtete Gas von ca. 240°C auf 315°C erwärmen. Die Wärmedurchgangszahl (k-Zahl) wird wie in Beispiel 1 auch einen Wert von ca. 160 W/m^{2°}C einnehmen. Führt man das heiße und kalte Gas im Gleichstrom durch den Wärmetauscher, so beträgt die niedrigste Rohrinnenwandtemperatur 315°C. Dadurch kann ein vorzeitiges und unerwünschtes desublimieren an Melamin sicher verhindert werden. Die sich daraus ergebende mittlere logarithmische Temperaturdifferenz beträgt ca. 73°C. Bei gleicher abzuführender Wärmemenge wie in Beispiel 1 benötigt man einen Wärmetauscher, der eine um den Faktor 1,8 kleinere Fläche benötigt. Es wird nunmehr eine geringere Wärmemenge von außen dem System zugeführt. Die eingesparte Wärmemenge von ca. 1800 kW entspricht einer jährlichen Einsparung von ca. 130000 Euro (mit 8000 h pro Jahr Betriebslaufzeit und 2,5 Euro pro GJ Brennstoff) oder entspricht einer Einsparung von etwa 12% an Primärbrennstoff.

### Bezugszeichenliste

a) Wirbelschichtreaktor
b) Heizelement
c) Gasheizelement
d) Gaskühler
e) Gasfilter
f) Kristallisationsvorrichtung
g) Produktzyklon
h) Kreisgasgebläse
i) Harnstoffwäsche
j) Kühleinheit
k) Harnstoffvorratsbehälter
l) Pumpe
m) Tropfenabscheider
n) Kompressor

## Patentansprüche

1. Verfahren zur Herstellung von Melamin durch die Zersetzung von Harnstoff in einem Wirbelschichtreaktor bei dem das heiße Reaktionsgas in einem Kühler abgekühlt wird, **dadurch gekennzeichnet, dass** die hierbei gewonnene Wärme zur Vorheizung auf das für die Erzeugung der Wirbelschicht benötigte Fluidisierungsgas unmittelbar übertragen wird.

2. Verfahren nach Anspruch 1, **dadurch gekennzeichnet, dass** die Abkühlung des Reaktionsgases in einem Gaskühler von etwa 380-420°C auf 210-300°C erfolgt.

3. Verfahren nach den Ansprüchen 1 und 2, **dadurch gekennzeichnet, dass** es sich bei dem Gaskühler um einen röhrenförmigen Wärmetauscher und/oder einen plattenförmigen Wärmetauscher handelt.

4. Verfahren nach den Ansprüchen 1 bis 3, **dadurch gekennzeichnet**, das die gasförmigen Medien den Wärmetauscher im Gleichstrom- oder im Gegenstromprinzip durchströmen.

5. Verfahren nach Ansprüchen 1 bis 4, **dadurch gekennzeichnet**, das die Temperaturregelung durch eine Umführungsleitung (by-pass) erfolgt.

## Claims

1. A method for producing melamine by decomposition of urea in a fluidised bed reactor in which the hot reaction gas is cooled in a cooler, **characterised in that** the heat hereby obtained is transferred directly for preheating to the fluidising gas required to produce the fluidised bed.

2. The method according to claim 1, **characterised in that** the cooling of the reaction gas in a gas cooler takes place from about 380-420°C to 210-300°C.

3. The method according to claims 1 and 2, **characterised in that** the gas cooler comprises a tubular heat exchanger and/or a plate-shaped heat exchanger.

4. The method according to claims 1 to 3, **characterised in that** the gaseous media flow through the heat exchanger according to the parallel flow or counterflow principle.

5. The method according to claims 1 to 4, **characterised in that** the temperature regulation is effected by a by-pass.

## Revendications

1. Procédé de fabrication de mélamine par décomposition d'urée dans un réacteur à combustible fluidisé dans lequel le gaz de réaction est refroidi dans un refroidisseur, **caractérisé en ce que** la chaleur récupérée à cet effet est directement transmise pour le préchauffage sur le gaz de fluidisation nécessaire pour le création du lit fluidisé.

2. Procédé selon la revendication 1, **caractérisé en ce que** le refroidissement du gaz de réaction s'effectue dans un refroidisseur à gaz d'environ 380 à 240 °C à 210 à 300 °C.

3. Procédé selon les revendications 1 et 2, **caractérisé en ce que** le refroidisseur à gaz est un échangeur thermique tubulaire et/ou un échangeur thermique en plaque.

4. Procédé selon les revendications 1 à 3, **caractérisé en ce que** les milieux gazeux circulent à travers l'échangeur thermique selon le principe de l'écoulement à courant parallèle ou à contre-courant.

5. Procédé selon les revendications 1 à 4, **caractérisé en ce que** la régulation de la température s'effectue par un conduit de dérivation (conduite de dérivation).
